# EUROPEAN PATENT SPECIFICATION

(11) **EP 3 000 424 B1**
(45) Date of publication and mention of the grant of the patent: **04.07.2018**
(21) Application number: 14801616.5
(22) Date of filing: 02.05.2014
(51) Int. Cl.: A61B 18/00, A61B 17/32

(54) **ULTRASONIC TREATMENT DEVICE**
ULTRASCHALLBEHANDLUNGSVORRICHTUNG
DISPOSITIF DE TRAITEMENT PAR ULTRASONS

(30) Priority: 21.05.2013 US 201361825723 P
(43) Date of publication of application: 30.03.2016
(73) Proprietor: Olympus Corporation, Tokyo 192-8507 (JP)
(72) Inventor: AKAGANE, Tsunetaka, Tokyo 192-8507 (JP)
(74) Representative: Gunzelmann, Rainer
(86) International application number: PCT/JP2014/062197
(87) International publication number: WO 2014/188867

(56) References cited:
- EP-A1- 2 591 734
- WO-A1-2012/176735
- JP-A- H03 151 954
- JP-A- 2001 161 705
- JP-A- 2001 161 705
- JP-A- 2004 283 362
- US-A- 5 076 276

## Description

### Technical Field

The present invention relates to an ultrasonic treatment device to conduct an ultrasonic treatment including ultrasonic suction.

### Background Art

Jpn. Pat. Appln. KOKAI Publication No. 2005-137481 (Patent Literature 1) has disclosed an ultrasonic treatment device to conduct an ultrasonic treatment known as ultrasonic suction. This ultrasonic treatment device comprises a probe which transmits ultrasonic vibration from a proximal direction to a distal direction. The ultrasonic suction is conducted by using a probe distal end face of the ultrasonically vibrating probe, and conducted by using a physical phenomenon known as cavitation. Specifically, since the probe repeats tens of thousands of high-velocity vibrations per second by ultrasonic vibration, pressure periodically varies in the vicinity of the probe distal face of the probe. When the pressure in the vicinity of the probe distal face is lower than saturated vapor pressure for only a short time due to a pressure variation, small air bubbles (cavities) are generated in a liquid within a body cavity or in a liquid supplied from a liquid supply unit to the vicinity of a treatment position of a living tissue. The generated air bubbles disappear due to force that acts when the pressure in the vicinity of the probe distal face increases (compression). The above-described physical phenomenon is called a cavitation phenomenon. An inelastic living tissue such as a liver cell is shattered and emulsified by impact energy when the air bubbles disappear. Such an ultrasonic treatment device is provided with a suction path inside the probe along a longitudinal axis. The shattered and emulsified living tissue is suctioned and collected from a suction opening at the distal end of the probe through the suction path. The above-described operations are continued to resect the living tissue. In this case, an elastic living tissue such as a blood vessel absorbs the impact and is therefore not easily shattered, so that living tissues are selectively shattered.

Document US 5 076 276 A discloses an ultrasound type treatment apparatus including an ultrasonic oscillation device for outputting an ultrasonic oscillation wave, a drive circuit for driving the ultrasonic oscillation device, a probe connected to the ultrasonic oscillation device to transmit that ultrasonic oscillation wave to an affected region of interest of a human being, a sheath formed around the probe, through which cooling medium is passed and a supply tank for supplying cooling medium to the sheath. In this treatment apparatus, an arithmetic operation circuit monitors a drive state of the ultrasonic oscillation device, a comparator is provided for receiving monitor information from the arithmetic operation circuit and for determining whether or not the supply of cooling medium to the sheath is normal and a controller is provided for stopping a drive of the ultrasonic oscillation device when the supply of the cooling medium is determined by the comparator as being not normal.

Document EP 2 591 734 A1 discloses a medical liquid supply device including a path defining portion which defines a communication path configured to allow communication between a liquid supply path and a suction path, a liquid supply unit configured to cause a liquid to flow into the liquid supply path in a flow amount greater than or equal to a standard flow amount so that the liquid supply path constantly has no parts lacking the liquid up to the communication path in a liquid supply direction, and a suction unit configured to perform suction via the suction path. The medical liquid supply device includes a control unit configured to control an amount of suction by the suction unit in accordance with a switching operation in a liquid supply mode input section to adjust a flow amount of the liquid flowing from the liquid supply path into the suction path via the communication path between a liquid-supply ON mode and a liquid-supply OFF mode.
Document JP 2001 161705 A discloses an ultrasonic treatment device for treating a biological tissue with ultrasonic vibrations. The ultrasonic treatment device comprises a main body for driving/controlling, a hand piece connected to the main body for generating ultrasonic vibrations, a probe connected to the hand piece for giving ultrasonic vibrations to the biological tissue, and a sheath for covering the probe except for the tip of the probe. The sheath consists of an inner sheath and an outer sheath. The inner diameter of the inner sheath is larger than the outer diameter of the probe, and a projection is formed at least in a part of the lumen of the inner sheath so that the inner diameter of the inner sheath is narrowed. The part including the projection is detachable from the outer sheath.

Document JP 2004 283362 A discloses an ultrasonic treatment device. The ultrasonic treatment device is provided with an ultrasonic treatment tool comprising a vibration transmission member for transmitting ultrasonic vibrations generated in an ultrasonic vibrator to a tip side, an ultrasonic probe connected to the tip side of a vibration transmission member for treating biological tissues by the ultrasonic vibrations transmitted from the vibration transmission member, and an insertion part jacket tube covering the vibration transmission member. For the vibration transmission member, a flange part is formed at the node position of the vibrations. The insertion part jacket tube is constituted of an exterior sheath and a tubular operation rod as an inner sheath inserted to the exterior sheath. A PTFE tube is fitted as an elastic tube to the inner surface of the tubular operation rod, the PTFE tube is abutted on the flange part, and the vibration transmission member is supported.

### Summary of Invention

An ultrasonic treatment device is defined by independent claim 1. The ultrasonic treatment device may comprise a vibration transmitting portion which extends along a longitudinal axis and which transmits ultrasonic vibration from a proximal direction to a distal direction, an inner surface which defines an internal space along the longitudinal axis inside the vibration transmitting portion, a first tube which extends along the longitudinal axis in the internal space, a path portion which is provided on the inner circumferential side of the first tube and through which a suction target to be suctioned passes toward the proximal direction, and a first spacer which intervenes between the inner surface and the first tube at a node position of the ultrasonic vibration in a longitudinal axis direction parallel to the longitudinal axis and which supports the first tube so that the outer circumferential surface of the first tube does not contact the inner surface.

### Technical Problem

An ultrasonic treatment device which prevents clogging with a treated fragment is provided.

### Brief Description of Drawings

FIG. 1 is a schematic diagram showing an ultrasonic treatment device according to a first example;
FIG. 2 is a sectional view schematically showing the configuration of a vibrator unit according to the first example;
FIG. 3 is a perspective view schematically showing an ultrasonic probe according to the first example;
FIG. 4 is a sectional view showing the vicinity of the distal end of the probe shown in FIG. 3 cut in a section parallel to a longitudinal axis;
FIG. 5 is a sectional view schematically showing the internal configuration of a sheath, and a coupling configuration of the sheath to a vibrator case according to the first example;
FIG. 6 is a sectional view schematically showing the configurations of the distal end of the probe and the distal end of the sheath according to the first example;
FIG. 7 is a sectional view showing the probe shown in FIG. 3 cut in the section parallel to the longitudinal axis;
FIG. 8 is a sectional view showing the probe shown in FIG. 7 cut in the section perpendicular to the longitudinal axis at a node position;
FIG. 9 is a sectional view showing the probe of the ultrasonic treatment device according to a modification of the first example cut in the section perpendicular to the longitudinal axis at the node position;
FIG. 10 is a sectional view showing a probe of an ultrasonic treatment device according to a second example cut in a section parallel to a longitudinal axis;
FIG. 11 is a sectional view showing a probe of an ultrasonic treatment device according to an embodiment of the present invention cut in a section parallel to a longitudinal axis;
FIG. 12 is a sectional view showing a probe of an ultrasonic treatment device according to a third example cut in a section parallel to a longitudinal axis;
FIG. 13 is a sectional view showing the probe according to a first modification of the third example cut in a section parallel to the longitudinal axis; and
FIG. 14 is a sectional view showing the probe according to a second modification of the third example cut in a section parallel to the longitudinal axis.

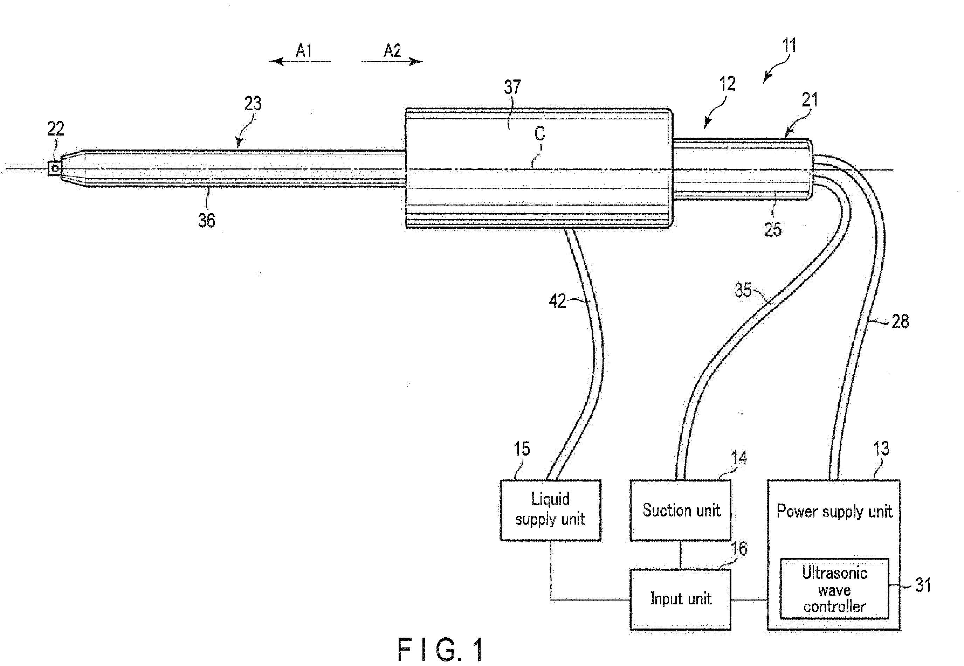

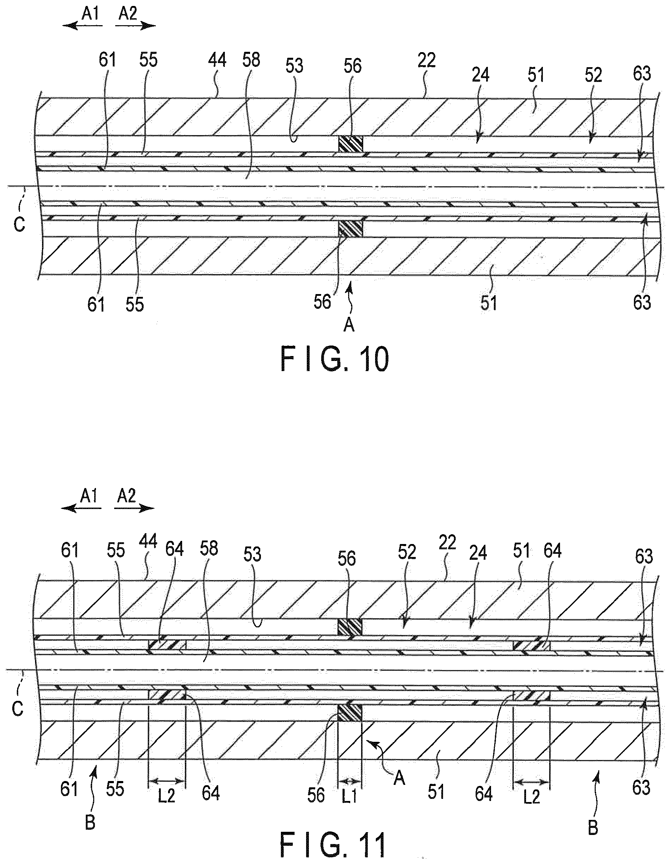

Figure 11 shows an embodiment of the invention. Figures 1-10 and 12-14 show exemplary ultrasonic treatment devices.

### DETAILED DESCRIPTION OF THE INVENTION

### (First example)

A first example is described with reference to FIG. 1 to FIG. 8. An ultrasonic treatment device 11 according to the present example is an ultrasonic suction device which selectively shatters and emulsifies a living tissue by using cavitation caused by ultrasonic vibration, and suctions a suction target such as the shattered and emulsified living tissue. The ultrasonic treatment device 11 has a longitudinal axis C. One of directions parallel to the longitudinal axis C is a distal direction (direction of an arrow A1 in FIG. 1), and the other direction parallel to the longitudinal axis C is a proximal direction (direction of an arrow A2 in FIG. 1).

As shown in FIG. 1, the ultrasonic treatment device 11 comprises an ultrasonic treatment instrument 12, a power supply unit 13, a suction unit 14, a liquid supply unit 15, and an input unit 16. The suction unit 14 and the liquid supply unit 15 are connected to the input unit 16. As shown in FIG. 1 to FIG. 6, the ultrasonic treatment instrument 12 comprises a vibrator unit 21, a probe 22, and a sheath unit 23.

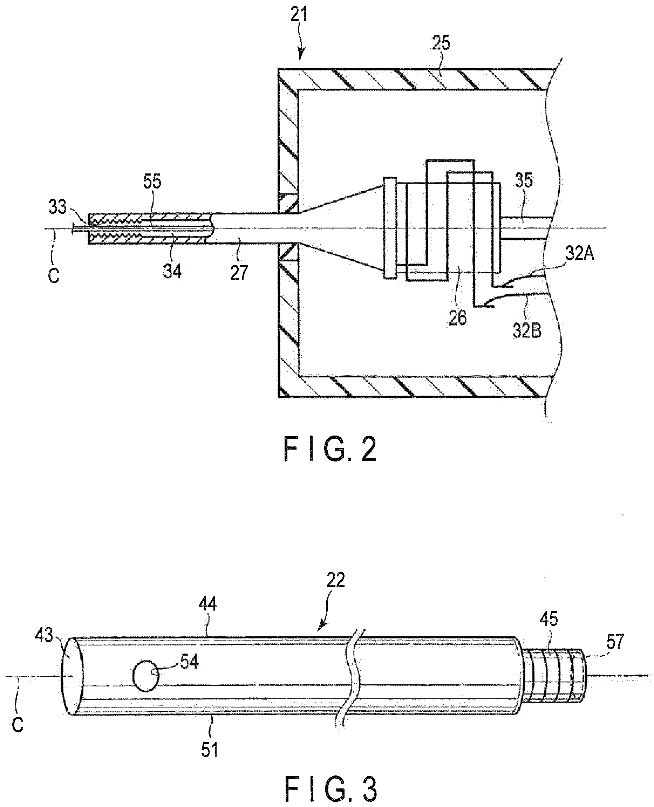

FIG. 2 is a diagram showing the configuration of the vibrator unit 21. The vibrator unit 21 comprises a vibrator case 25, an ultrasonic vibrator 26, and a horn 27. One end of a cable 28 is connected to the proximal end of the vibrator case 25. The other end of the cable 28 is connected to a power supply unit 13. The power supply unit 13 comprises an ultrasonic wave controller 31. An input unit 16 such as a foot switch is connected to the power supply unit 13.

As shown in FIG. 2, the ultrasonic vibrator 26 is provided inside the vibrator case 25. The ultrasonic vibrator 26 comprises a piezoelectric element to convert an electric current into ultrasonic vibration. One end of each of electric signal lines 32A and 32B is connected to the ultrasonic vibrator 26. Each of the electric signal lines 32A and 32B has the other end connected to the ultrasonic wave controller 31 of the power supply unit 13 through the cable 28. Ultrasonic vibration is generated in the ultrasonic vibrator 26 by the supply of an electric current to the ultrasonic vibrator 26 from the ultrasonic wave controller 31 via the electric signal lines 32A and 32B. An ultrasonic wave generating unit which generates ultrasonic vibration is formed by the vibrator unit 21 and the power supply unit 13.

A horn 27 which increases the amplitude of the ultrasonic vibration is coupled to the distal-direction side of the ultrasonic vibrator 26. The horn 27 is attached to the vibrator case 25. An internal thread portion 33 is formed at the distal end of the inner circumferential surface of the horn 27. A cavity portion 34 is formed in the ultrasonic vibrator 26 and the horn 27 along the longitudinal axis C. A later-described first tube 55 extends into the horn. One end of a suction tube 35 is connected to the cavity portion 34. As shown in FIG. 1, the suction tube 35 extends to the outside of the vibrator case 25, and is connected at the other end to the suction unit 14.

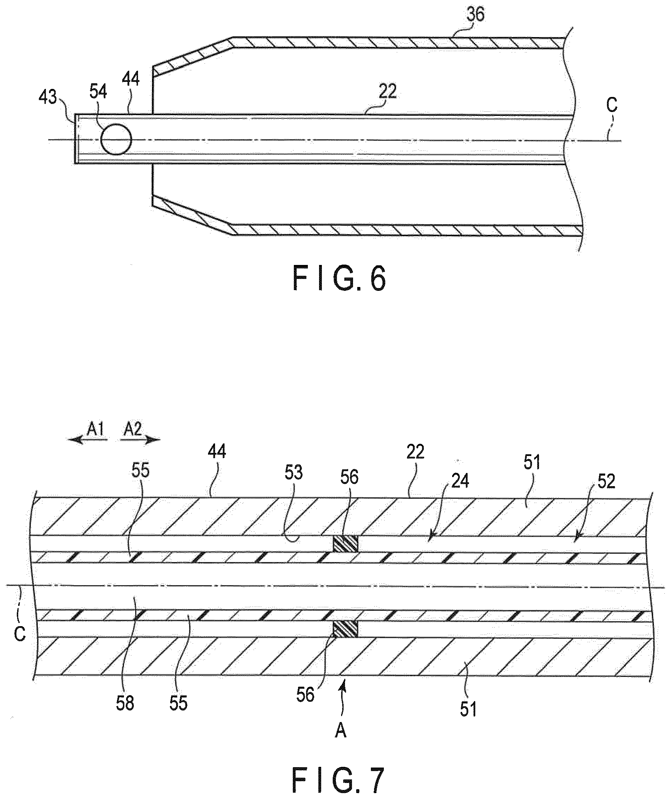

As shown in FIG. 1, the sheath unit 23 comprises a sheath 36 for protecting the probe 22, and a holding case 37 to be held by a surgeon. The sheath 36 extends along the longitudinal axis C, and the probe 22 is inserted through the sheath 36. As shown in FIG. 6, the probe 22 is inserted through the sheath 36 so that a probe distal face 43 is located closer to the distal-direction side than the distal end of the sheath 36. In other words, the probe distal face 43 of the probe 22 inserted through the sheath 36 is not covered with the sheath 36 and is exposed. The sheath 36 is inserted into the holding case 37 from the distal-direction side, and the vibrator unit 21 is inserted into the holding case 37 from the proximal-direction side. The sheath 36 and the vibrator case 25 are coupled to each other inside the holding case 37.

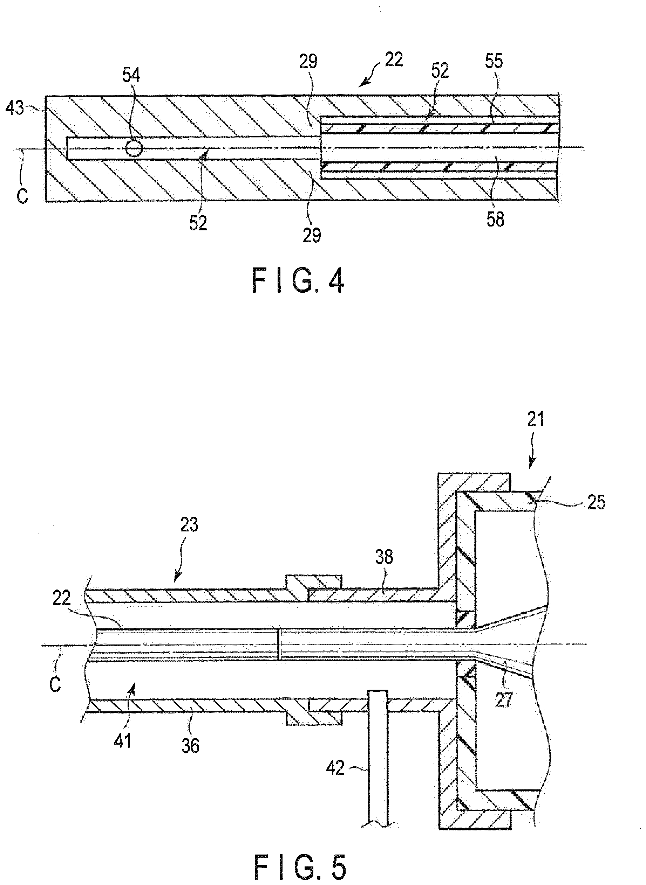

As shown in FIG. 5, a clearance 41 is provided between the inner circumferential portion of the sheath 36 and the outer circumferential portion of the probe 22. The clearance 41 is provided from the distal end of the sheath 36 along the longitudinal axis C between the inner circumferential portion of the sheath 36 and the outer circumferential portion of the probe 22. The distal end of a cylindrical intermediary member 38 is attached to the proximal end of the sheath 36. The distal end of the vibrator case 25 is attached to the proximal end of the intermediary member 38. The sheath 36 may be attachable to and detachable from the intermediary member 38 by, for example, screw tightening.

As shown in FIG. 5, the clearance 41 extends to the distal face of the vibrator case 25. One end of a liquid supply tube 42 is connected to the intermediary member 38. The inside of the liquid supply tube 42 is in communication with the clearance 41. As shown in FIG. 1, the liquid supply tube 42 extends to the outside of the holding case 37, and is connected to the liquid supply unit 15. The liquid supply unit 15 is connected to the input unit 16. When the liquid supply unit 15 is driven by, for example, an input in the input unit 16, a liquid such as physiological saline passes through the liquid supply tube 42 and the clearance 41 in order. The liquid is then supplied to, for example, a living tissue from the distal end of the clearance 41.

In ultrasonic suction, ultrasonic vibration is transmitted to the probe distal face 43 located at an antinode position of ultrasonic vibration. At the same time, a cavitation phenomenon is caused by the liquid supply through the clearance 41. This liquid supply may be performed in a treatment other than the ultrasonic suction treatment. For example, a bleeding part may be checked, or a body cavity may be washed by the liquid supply.

The probe 22 is made of a metallic material such as a titanium alloy. The material of the probe 22 is not limited to the titanium alloy, and may be some other metallic material such as duralumin.

As shown in FIG. 3, the probe 22 extends along the longitudinal axis C. The probe 22 comprises the above-mentioned probe distal face 43, and a probe outer circumferential portion 44. An external thread portion 45 which is screwed to the internal thread portion 33 of the horn 27 is provided at the proximal end of the probe outer circumferential portion 44. The external thread portion 45 is screwed to the internal thread portion 33, and the probe 22 is thereby attached to the distal-direction side of the horn 27. When the probe 22 is connected to the horn 27, the ultrasonic vibration generated in the ultrasonic vibrator 26 is transmitted to the probe distal face 43 via the horn 27 and the probe 22. When the ultrasonic vibration is transmitted to the probe distal face 43 and the above-mentioned liquid supply is performed, the living tissue is shattered and emulsified in the probe distal face 43 by the use of the cavitation phenomenon. By the cavitation phenomenon, a tissue having high elasticity such as a blood vessel is not shattered, and an inelastic living tissue such as a liver cell is selectively shattered and emulsified.

The length of a combination of the probe 22, the ultrasonic vibrator 26, and the horn 27 along the longitudinal axis C is set so that the probe distal face 43 of the probe 22 is located at the antinode position of the ultrasonic vibration, and so that the proximal end of the ultrasonic vibrator 26 is located at the antinode position of the ultrasonic vibration. When the probe distal face 43 is located at the antinode position of the ultrasonic vibration, the cavitation is more efficiently caused. The ultrasonic vibration is longitudinal vibration having a vibration transmission direction and a vibration direction parallel to each other, and the vibration transmission direction and the vibration direction are parallel to the longitudinal axis C. The probe 22 and the horn 27 constitute a vibration transmission portion which transmits the ultrasonic vibration of the ultrasonic vibrator 26 from the proximal direction to the distal direction.

As shown in FIG. 3 and FIG. 7, the probe 22 has a main body 51 which conducts a treatment by the ultrasonic vibration transmitted from the ultrasonic vibrator 26, an internal space 52 inside the main body 51, and a space defining surface 53 (inner surface) which defines the internal space 52. The main body 51 includes the above-mentioned probe distal face 43 and the probe outer circumferential portion 44. The internal space 52 is provided along the longitudinal axis C from the distal end of the probe 22. As shown in FIG. 3, two openings 54, which are in communication with the internal space 52, are provided in the probe outer circumferential portion 44 of the probe 22 apart from each other by an angular position of about 180° around the longitudinal axis. The two openings 54 are provided in the vicinity of the probe distal face 43.

As shown in FIG. 7, a tube unit 24 is provided in the internal space 52. The tube unit 24 has the first tube 55 provided along the longitudinal axis C, and a first spacer 56 intervening between the space defining surface 53 (inner surface) of the probe 22 and the outer circumferential surface of the first tube 55. A suction path to pass a suction target such as the living tissue shattered and emulsified by the cavitation phenomenon and the supplied liquid is provided inside the first tube 55.

The first tube 55 is formed into an elongated circular cylindrical shape by a synthetic resin material such as PTFE. Thus, the inner circumferential surface of the first tube 55 has lower frictional properties (higher slipperiness) than the space defining surface 53 of the probe 22. The first tube 55 has lower heat conductivity than the probe 22. A path portion 58, through which a suction target such as the living tissue to be suctioned passes toward the proximal direction, is provided on the inner circumferential side of the first tube 55. The first tube 55 is watertightly connected to the internal space 52, which is in communication with the two openings 54 of the probe 22 on the distal-direction side. Specifically, as shown in FIG. 4, the end of the first tube 55 on the distal-direction side is bonded to a step portion 29 provided inside from the probe 22 by, for example, an adhesive agent. The end of the first tube 55 on the proximal direction side is also bonded to a step portion provided inside the cavity portion 34 of the horn 27 by, for example, an adhesive agent in the same manner as the end on the distal-direction side. Although the first tube 55 is connected to the cavity portion 34 of the horn 27 in the proximal direction, the first tube 55 may be directly connected to the suction tube 35. The first tube 55 may be configured to be attachable to and detachable from the internal space 52 of the probe 22 via an opening portion 57 (see FIG. 3) on the proximal side of the probe 22. The first tube 55 may also be configured to be attachable to and detachable from the cavity portion 34 of the horn 27.

The first spacer 56 (lining) is made of a synthetic resin material such as polytetrafluoroethylene (PTFE). Thus, the first spacer 56 has lower heat conductivity than the probe 22. The first spacer 56 is formed into a cylindrical shape (circular cylindrical shape) around the first tube 55 in the present example. The first spacer 56 supports the first tube 55 so that the outer circumferential surface of the first tube 55 does not contact the space defining surface 53 (inner surface).

The first spacer 56 is provided at a position corresponding to one node position A of the ultrasonic vibration generated by the ultrasonic vibrator 26 in the longitudinal axis direction. That is, the length of the first spacer 56 in the longitudinal axis C direction is smaller than the entire length of the probe 22 in the longitudinal axis C direction. At the node position of the ultrasonic vibration where amplitude becomes zero (i.e. no vibration is generated), stress by the ultrasonic vibration is greater. Thus, at the node position of the ultrasonic vibration, heat is generated by internal friction of the metal that forms the probe.

Next, the function of the ultrasonic treatment device 11 according to the present example is described. When a living tissue is ultrasonically suctioned by the use of the ultrasonic treatment device 1, an electric current is supplied to the ultrasonic vibrator 26 from the ultrasonic wave controller 31 via the electric signal lines 32 by, for example, an operation in the input unit 16. As a result, ultrasonic vibration is generated in the ultrasonic vibrator 26. The ultrasonic vibration is transmitted to the probe distal face 43 of the probe 22. A liquid such as physiological saline is supplied to the living tissue through the clearance 41 by the liquid supply unit 15. Cavitation is caused by the transmission of the ultrasonic vibration to the probe distal face 43 and by the supply of the liquid. A living tissue having low elasticity such as a liver cell is selectively shattered and resected (removed) as a treated fragment (a fragment of living tissue) by the cavitation. At the same time, high-temperature heat is generated by the internal friction resulting from the ultrasonic vibration at the node position A of the probe 22.

The suction unit 14 applies negative pressure to the suction path constituted by the first tube 55, the cavity portion 34, and the suction tube 35, and suctions the resected suction target (treated fragment) via the openings 54. The suction target is suctioned to the suction unit 14 through the first tube 55, the cavity portion 34, and the suction tube 35, and thus collected. In this instance, the suction target (treated fragment) is collected through the first tube 55 without direct contact with the probe 22 that is generating heat.

According to the first example, the ultrasonic treatment device 11 comprises a vibration transmitting portion which extends along the longitudinal axis C and which transmits ultrasonic vibration from a proximal direction to a distal direction, the space defining surface 53 (inner surface) which defines the internal space 52 along the longitudinal axis C inside the vibration transmitting portion, the first tube 55 which extends in the internal space 52 along the longitudinal axis C, the path portion 58 which is provided on the inner circumferential side of the first tube 55 and through which a suction target to be suctioned passes toward the proximal direction, and a first spacer which intervenes between the space defining surface and the first tube at one node position of the ultrasonic vibration in a longitudinal axis direction parallel to the longitudinal axis and which supports the first tube so that the outer circumferential surface of the first tube does not contact the space defining surface.

In general, in the ultrasonic treatment device 11, at the position corresponding to the node position A of the ultrasonic vibration in the probe 22, no vibration is generated, but the temperature becomes high due to, for example, the internal friction. Meanwhile, no vibration occurs at the node position A of the ultrasonic vibration, so that treated fragments flowing through the internal space 52 tend to be accumulated in a part corresponding to the node position A. In a conventional structure, the treatment fragments thus accumulated may coagulate and adhere to the space defining surface 53 of the high-temperature probe 22, and subsequent treatment fragments may accumulate on the former adhering treatment fragments and clog the internal space 52.

According to the configuration described above, the first tube 55 and the first spacer 56 intervene between the treatment fragments and the space defining surface 53 of the probe 22, and the first tube 55 contacts the probe 22 via the first spacer 56. Thus, even when the temperature of the probe 22 has become high at the node position A of the ultrasonic vibration, the efficiency of the transmission of the heat at the node position A to the first tube 55 can be degraded (i.e. the heat transmission to the first tube 55 is difficult).

The first spacer 56 and the first tube 55 are lower in heat conductivity than the probe 22. Thus, the transfer of heat to the first tube 55 is difficult, and the coagulation of the treatment fragments and the adhesion of the coagulated treatment fragments to the first tube 55 can be prevented.

Furthermore, the inner surface of the first tube 55 has higher slipperiness than the space defining surface 53 (inner surface) of the probe 22. According to this configuration, the adhesion of the treatment fragments to the first tube 55 and the clogging of the first tube 55 after the adhesion can be effectively prevented. Since the first tube 55 can be attached to and detached from the inside of the internal space 52 of the probe 22, the ultrasonic treatment device 11 can quickly become reusable by the replacement of the first tube 55 even if the first tube 55 is clogged.

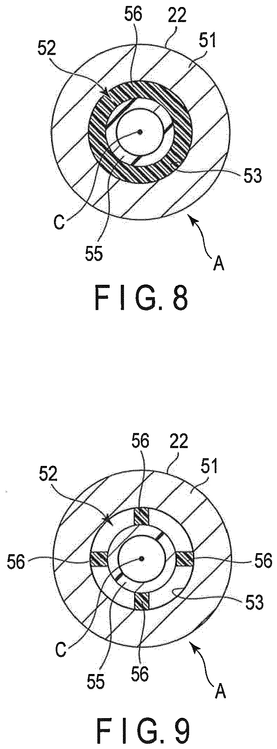

A modification of the ultrasonic treatment device 11 according to the first example is described with reference to FIG. 9. In this modification, the shape of the first spacer 56 is not cylindrical as in the first example described above, but is discontinuously formed (i.e. discontinuously formed in the circumferential direction of the first tube 55) in a cut section of the probe 22 that intersects (at right angles) with the longitudinal axis C as shown in FIG. 9. It can also be said that the first spacer 56 abuts on the outer circumferential portion of the first tube 55 in part of a range in the direction around the longitudinal axis C.

In this modification, the first spacer 56 is provided to be separated (divided) at four places; for example, the upper side, lower side, right side, and left side of the first tube 55. Thus, the area (sectional area) of the section of the first spacer 56 that intersects with the longitudinal axis C can be smaller. Therefore, even when the temperature of the probe 22 has become high at the node position A of the ultrasonic vibration, the efficiency of the transmission of the heat at the node position A to the first tube 55 can be degraded (i.e. the heat transmission to the first tube 55 is difficult). As a result, the coagulation of the treatment fragments and the adhesion of the coagulated treatment fragments to the first tube 55 can be further prevented. The first spacer 56 is not exclusively disposed in the above-mentioned manner, and may be disposed to be separated (divided) into six or eight places.

### (Second example)

Next, a second example is described with reference to FIG. 10. An ultrasonic treatment device 11 according to the second example is the following modification of the configuration according to the first example. The main differences between the first example and the second example are explained, and an explanation of the parts that are the same as those in the first example is omitted.

As shown in FIG. 10, the tube unit 24 is provided in the internal space 52 of the probe 22. The tube unit 24 has the first tube 55 provided along the longitudinal axis C, the first spacer 56 intervening between the space defining surface 53 (inner surface) of the probe 22 and the first tube 55, and a second tube 61 provided on the inner circumferential side of the first tube 55 along the longitudinal axis C. The configurations of the first tube 55 and the first spacer 56 are similar to those in the first example. The second tube 61 is disposed inside the first tube 55. Thus, the second tube 61 has a diameter smaller than the diameter of the first tube 55. The second tube 61 is formed into an elongated circular cylindrical shape by a synthetic resin material such as PTFE. Thus, the inner circumferential surface of the second tube 61 has lower frictional properties (higher slipperiness) than the space defining surface 53 of the probe 22. The second tube 61 has lower heat conductivity than the probe 22. The path portion 58 through which a suction target to be suctioned passes toward the proximal direction is formed inside the second tube 61. The suction target which has been treated with the ultrasonic vibration can pass through this path portion 58.

The second tube 61 is watertightly connected to the internal space 52 which is in communication with the two openings 54 of the probe 22 on the distal-direction side as in the structure of the distal-direction end of the first tube 55 according to the first example. The proximal-direction end of the first tube 55 is also bonded to a step portion protruding inside the cavity portion 34 of the horn 27 by, for example, an adhesive agent in the same manner as the distal-direction side end. Although the second tube 61 is connected to the cavity portion 34 of the horn 27 in the proximal direction, the second tube 61 may be directly connected to the suction tube 35. The second tube 61 may be configured to be attachable to and detachable from the internal space 52 of the probe 22 via the opening portion 57 on the proximal side of the probe 22. The second tube 61 may also be configured to be attachable to and detachable from the cavity portion 34 of the horn 27.

A second clearance 63 (air layer, clearance), which in effect serves as a heat insulating material between the first tube 55 and the second tube 61, is provided between the inner circumferential surface of the first tube 55 and the outer circumferential surface of the second tube 61.

Next, the function of the ultrasonic treatment device 11 according to the present example is described. In response to, for example, an operation in the input unit 16, an electric current is supplied to the ultrasonic vibrator 26, and ultrasonic vibration is generated in the ultrasonic vibrator 26. The ultrasonic vibration is then transmitted to the probe distal face 43 of the probe 22. A liquid such as physiological saline is supplied to the living tissue by the liquid supply unit 15. Cavitation is caused by the probe distal face 43, so that a living tissue having low elasticity is selectively shattered and resected as a treated fragment (a fragment of living tissue). At the same time, high-temperature heat is generated by the internal friction resulting from the ultrasonic vibration at the node position A of the probe 22.

The suction unit 14 applies negative pressure to the suction path constituted by the second tube 61, the cavity portion 34, and the suction tube 35, and suctions the resected suction target (treated fragment) via the openings 54. The suction target is suctioned to the suction unit 14 through the second tube 61, the cavity portion 34, and the suction tube 35, and thus collected. In this instance, the suction target (treatment fragment) is collected through the second tube 61 without direct contact with the probe 22 that is generating heat, so that the adhesion of the treatment fragments (fragments of living tissue) to the space defining surface 53 of the probe 22 can be prevented.

According to the present example, the ultrasonic treatment device 11 further comprises the second tube 61 which extends along the longitudinal axis C on the inner circumferential side of the first tube 55 and in which the path portion 58 is formed. According to this configuration, the second tube 61 is further provided inside the first tube 55, to enable making the transmission of the heat on the side of the probe 22 to the second tube 61 more difficult. As a result, the adhesion of the treatment fragments to the second tube 61 can be prevented, and the formation of a source of clogging in the second tube 61 can be prevented. When the second clearance 63 (air layer) is provided between the first tube 55 and the second tube 61, the second clearance 63 in effect serves as a heat insulating material, to make the transmission of the heat on the side of the first tube 55 to the side of the second tube 61 difficult. This can also prevent the adhesion of the treatment fragments to the second tube 61.

The second tube 61 is lower in heat conductivity than the probe 22. Thus, the transfer of heat to the second tube 61 is difficult, and the coagulation of the treatment fragments and the adhesion of the coagulated treatment fragments to the second tube 61 can be prevented.

Furthermore, the inner surface of the second tube 61 has higher slipperiness than the space defining surface 53 of the probe 22. According to this configuration, the coagulation of the treatment fragments and the adhesion of the coagulated treatment fragments to the second tube 61 can be effectively prevented. Since the second tube 61 can be attached to and detached from the inside of the internal space 52 of the probe 22, the ultrasonic treatment device 11 can quickly become reusable by the replacement of the second tube 61 even if the second tube 61 is clogged.

### (Embodiment of the present invention)

Next, an embodiment of the present invention is described with reference to FIG. 11. An ultrasonic treatment device 11 according to the embodiment is the following modification of the configuration according to the second example. The main differences between the second example and the embodiment are explained, and an explanation of the parts that are the same as those in the second example is omitted.

As shown in FIG. 11, the tube unit 24 is provided in the internal space 52 of the probe 22. The tube unit 24 has the first tube 55 provided along the longitudinal axis C, the first spacer 56 intervening between the space defining surface 53 (inner surface) of the probe 22 and the first tube 55, the second tube 61 provided inside the first tube 55, and a second spacer 64 intervening between the first tube 55 and the second tube 61. The configurations of the first tube 55, the first spacer 56, and the second tube 61 are similar to those in the first example and the second example.

The second clearance 63 (air layer, clearance), which in effect serves as a heat insulating material between the first tube 55 and the second tube 61, is provided between the inner circumferential surface of the first tube 55 and the outer circumferential surface of the second tube 61. In the present embodiment, the second spacer 64 intervenes between the first tube 55 and the second tube 61, so that the second clearance 63, which always has a constant size, is ensured regardless of the placement angle (use angle) of the probe 22.

The second spacer 64 (second lining) intervenes between the outer circumferential surface of the first tube 55 and the inner circumferential surface of the second tube 61. The second spacer 64 supports the second tube 61 so that the outer circumferential surface of the second tube 61 does not contact the inner circumferential surface of the first tube 55. The second spacer 64 is made of a synthetic resin material such as polytetrafluoroethylene (PTFE). Thus, the second spacer 64 has lower heat conductivity than the probe 22.

The second spacer 64 is formed into a cylindrical shape (circular cylindrical shape) around the first tube 55. The second spacer 64 is provided apart from (off) the node position A of the ultrasonic vibration generated by the ultrasonic vibrator 26 in the longitudinal axis C direction, and, for example, a pair of second spacers 64 is provided on both sides across the node position A. It can also be said that the second spacer 64 is provided at an antinode position B of the ultrasonic vibration generated by the ultrasonic vibrator 26. The number of the second spacers 64 is not exclusively two. For example, multiple second spacers 64 may be provided off the node position A of the ultrasonic vibration. In this case, a certain space is preferably provided between the second spacers 64. The second spacers 64 may be configured to be attachable to and detachable from the internal space of the probe 22 via the opening portion 57 on the proximal side of the probe 22.

In the present embodiment, a first axially parallel dimension L1 of the first spacer 56 in the longitudinal axis direction is smaller than a second axially parallel dimension L2 of the second spacer 64 in the longitudinal axis direction.

Next, the function of the ultrasonic treatment device according to the present embodiment is described. In response to, for example, an operation in the input unit 16, an electric current is supplied to the ultrasonic vibrator 26, and ultrasonic vibration is generated in the ultrasonic vibrator 26. The ultrasonic vibration is then transmitted to the probe distal face 43 of the probe 22. A liquid such as physiological saline is supplied to the living tissue by the liquid supply unit 15. Cavitation is caused by the probe distal face 43, so that a living tissue having low elasticity is selectively shattered and resected (removed) as a treatment fragment (a fragment of living tissue fragment). At the same time, the temperature becomes high due to the internal friction resulting from the ultrasonic vibration at the node position A of the probe 22.

The suction unit 14 applies negative pressure to the suction path constituted by the second tube 61, the cavity portion 34, and the suction tube 35, and suctions the resected suction target (treatment fragment) via the openings 54. The suction target is suctioned to the suction unit 14 through the second tube 61, the cavity portion 34, and the suction tube 35, and thus collected. In this instance, the suction target (treatment fragment) is collected through the second tube 61 without direct contact with the probe 22 that is generating heat. Since the second clearance 63 (air layer) is always ensured by the second spacers 64 between the first tube 55 and the second tube 61, the first tube 55 and the second tube 61 do not come into direct contact, and the transfer of heat between the first tube 55 and the second tube 61 is minimized.

According to the embodiment, the ultrasonic treatment device 11 further comprises the second spacer 64 which intervenes between the first tube 55 and the second tube 61 and which supports the second tube 61 so that the outer circumferential surface of the second tube 61 does not contact the inner circumferential surface of the first tube 55. According to this configuration, regardless of the use angle of the probe 22, contact between the first tube 55 and the second tube 61 is always prevented, and the second clearance 63 (air layer, clearance) is ensured between the first tube 55 and the second tube 61. Thus, the transfer of the heat generated in the probe 22 to the second tube 61 via the first tube 55 can be more effectively prevented. As a result, it is possible to further prevent the suction target (treated fragment) from adhering to the second tube 61 and becoming a source of clogging in the second tube.

In the present embodiment, the second spacer 64 is provided off the node position A of the ultrasonic vibration. According to this configuration, even if heat is transmitted to the second tube 61 from the probe 22 via the first spacer 56, the first tube 55, and the second spacer 64, the distance in which the heat reaches the second tube 61 can be longer. Thus, the heat at the probe 22 can be guided in a diverting manner, and the transfer of heat to the second tube 61 can be prevented as much as possible. As a result, it is possible to prevent the treatment fragments from adhering to the inside of the second tube 61 and clogging the second tube 61.

The second spacer 64 is lower in heat conductivity than the probe 22. Therefore, this enables making the transfer of heat to the second tube 61 difficult, and the adhesion of the treatment fragments to the second tube 61 can be prevented.

The inner surface of the second tube 61 has higher slipperiness than the space defining surface 53 of the probe 22. According to this configuration, the adhesion of the treated fragments to the second tube 61 can be effectively prevented. Since the second tube 61 can be attached to and detached from the inside of the internal space 52 of the probe 22, the ultrasonic treatment device 11 can quickly become reusable by the replacement of the second tube 61 even if the second tube 61 is clogged.

The first axially parallel dimension of the first spacer 56 in the longitudinal axis C direction is smaller than the second axially parallel dimension of the second spacer 64 in the longitudinal axis C direction. According to this configuration, the sectional area of the first spacer 56 in the longitudinal axis direction can be smaller, and this enables making the transfer of heat to the first tube 55 from the probe 22in the first spacer 56 close to the heat generation source difficult.

### (Third example)

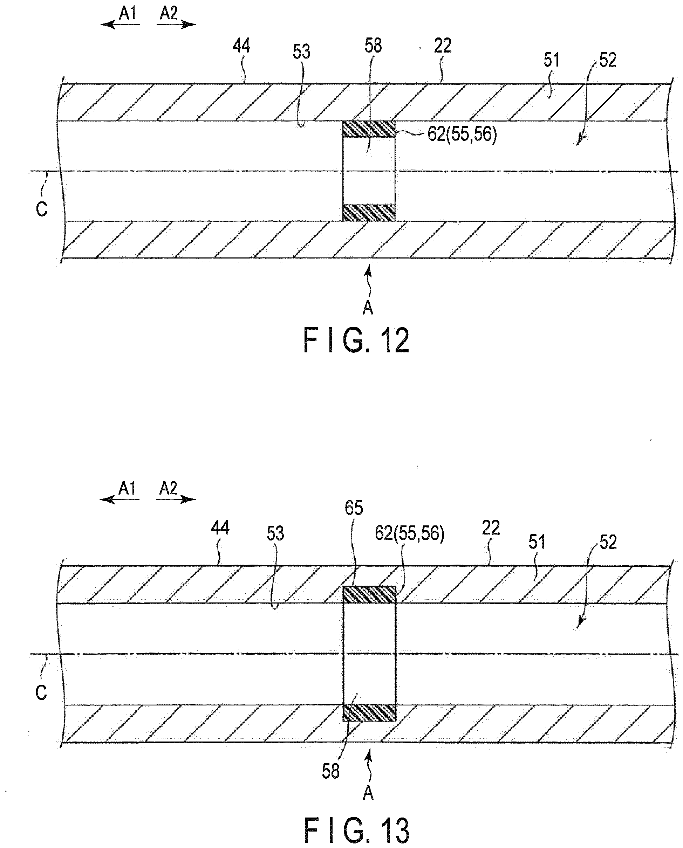

Next, a third example is described with reference to FIG. 12. An ultrasonic treatment device 11 according to the third example is the following modification of the configuration according to the first example. The main differences between the third example and the first example are explained, and an explanation of the parts that are the same as those in the first example is omitted.

As shown in FIG. 12, the tube unit 24 is provided in the internal space 52 of the probe 22. The tube unit 24 comprises a ring member 62 that integrates the first tube 55 according to the first example with the first spacer 56 according to the first example. The ring member 62 is in circumferential abutment with the space defining surface 53 (inner surface) in a direction around the longitudinal axis C, and is in close contact with the space defining surface 53 of the probe 22. Thus, the suction target (treated fragment, living tissue fragment) does not enter the space between the ring member 62 and the space defining surface 53 of the probe 22.

The ring member 62 is formed into an elongated circular cylindrical shape by a synthetic resin material such as PTFE. Thus, the ring member 62 has lower frictional properties (higher slipperiness) than the space defining surface 53 of the probe 22. The ring member 62 has lower heat conductivity than the probe 22. The ring member 62 is provided at the position corresponding to the node position A of the ultrasonic vibration. The path portion 58 through which the suction target (treatment fragment, living tissue fragment) passes toward the proximal direction, and the inner circumferential side, which defines the path portion 58, are provided inside the ring member 62.

The ring member 62 is provided to include the node position A where the first spacer 56 is located in the longitudinal axis C direction. The ring member 62 is provided over a predetermined range smaller than a quarter wavelength of the ultrasonic vibration.

The ring member 62 may be configured to be attachable to and detachable from the internal space of the probe 22 via the opening portion 57 on the proximal side of the probe 22.

Next, the function of the ultrasonic treatment device 11 according to the present example is described. In response to, for example, an operation in the input unit 16, an electric current is supplied to the ultrasonic vibrator 26, and ultrasonic vibration is generated in the ultrasonic vibrator 26. The ultrasonic vibration is then transmitted to the probe distal face 43 of the probe 22. A liquid such as physiological saline is supplied to the living tissue by the liquid supply unit 15. Cavitation is caused by the probe distal face 43, so that a living tissue having low elasticity is selectively shattered and resected as a treated fragment (a fragment of living tissue). At the same time, the temperature becomes high due to the internal friction resulting from the ultrasonic vibration at the node position A of the probe 22.

The suction unit 14 applies negative pressure to the suction path constituted by the internal space 52 of the probe 22, the ring member 62, the cavity portion 34, and the suction tube 35, and suctions the resected suction target (treatment fragment) via the openings 54. The suction target is suctioned to the suction unit 14 through the internal space 52 of the probe 22, the ring member 62, the cavity portion 34, and the suction tube 35, and is thus collected. In particular, in the vicinity of the node position A of the ultrasonic vibration, the suction target (fragment, treated fragment) flows through the ring member 62 without direct contact with the space defining surface 53 of the probe 22 that is generating heat. Thus, the coagulation of the suction target on the space defining surface 53 of the probe 22 in the vicinity of the node position A of the ultrasonic vibration is prevented.

According to the present example, the ultrasonic treatment device 11 further comprises the ring member 62 which is formed by the integration of the first tube 55 and the first spacer 56 and which abuts on the space defining surface 53 circumferentially in the direction around the longitudinal axis C and in which the path portion 58 is formed. According to this configuration, the number of components can be reduced, and the configuration of the ultrasonic treatment device 11 can be simplified. Moreover, the entrance of the treatment fragments is prevented in the space between the space defining surface 53 of the probe 22 that reaches a high temperature due to the ultrasonic vibration, and the first tube 55 and the first spacer 56 that are integrated. Therefore, it is possible to prevent the treatment fragments from adhering to the space defining surface 53 of the probe 22 and becoming a source of clogging of the internal space 52.

The ring member 62 is provided over a predetermined range which includes the node position A where the first spacer 56 is located in the longitudinal axis C direction and which is smaller than a quarter wavelength of the ultrasonic vibration. In general, the temperature becomes high due to the internal friction resulting from the ultrasonic vibration at the node position A of the ultrasonic vibration in the probe 22. According to the configuration described above, it is possible to prevent the suction target from adhering to the space defining surface 53 of the probe 22 at the node position A of the probe 22 and becoming a source of clogging of the internal space 52. Moreover, the first tube 55 and the first spacer 56 that are integrally provided can have the necessary sizes or minimum lengths in the longitudinal axis C direction, and the manufacturing costs of the ultrasonic treatment device 11 can also be reduced.

The ring member 62 is lower in heat conductivity than the probe 22. According to this configuration, this enables making the transmission of the heat at the probe 22 to the first tube 55 and the first spacer 56 that are integrally provided difficult, so that the adhesion of the treatment fragments to the inner surface of the first tube 55 can be prevented.

The inner surface of the ring member 62 has higher slipperiness than the space defining surface 53 of the probe 22. According to this configuration, it is possible to prevent the treatment fragments from adhering to the inner surfaces of the first tube 55 and the first spacer 56 that are integrally provided and producing a source of clogging inside the first tube 55.

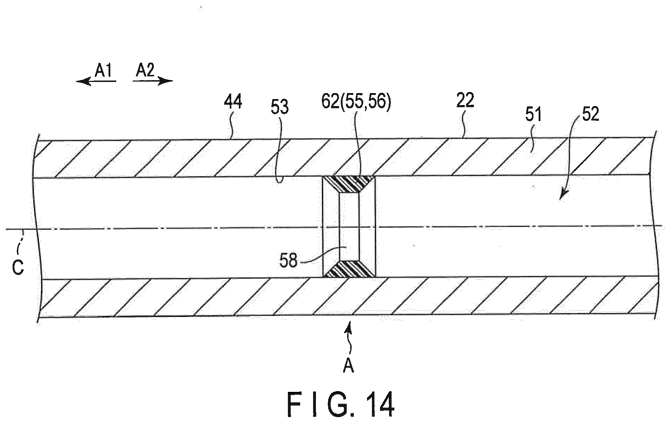

Although the flow path through which the treatment fragments flow is stepped between the space defining surface 53 of the probe 22 and the end of the ring member 62 in FIG. 12, a recess 65 may be provided in the space defining surface 53 of the probe 22 to eliminate the step, and the ring member 62 may be then disposed in the recess 65 as shown in FIG. 13 (a first modification of the third example). Alternatively, as shown in FIG. 14, the end faces of the ring member 62 may be oblique so that the inside diameter of the ring member 62 (the diameter of the inside cavity portion) increases closer to the ends in the longitudinal axis C direction (a second modification of the third example). As a result, it is difficult for the treatment fragments to accumulate on the boundary between the space defining surface 53 of the probe 22 and the ends of the ring member 62, and it is possible to effectively prevent these parts from becoming sources of clogging.

The material of the ring member 62 is not limited to PTFE, and may be some other kind of synthetic resin (e.g. rubber or some other resin material). In this case, the space defining surface 53 may be surface-treated to ensure the low frictional properties (slipperiness) of the space defining surface 53 of the ring member 62. The surface treatment includes, for example, coating such as fluorocarbon resin coating, and polishing.

The present invention is not limited to the embodiment described above. The ultrasonic treatment devices 11 according to the embodiment and examples can be combined into one ultrasonic treatment device 11.

## Claims

1. An ultrasonic treatment device (11) comprising:
an ultrasonic vibrator (26) generating ultrasonic vibration;
a horn (27) coupled to the ultrasonic vibrator (26);
a vibration transmitting portion which extends along a longitudinal axis (C) and which transmits the ultrasonic vibration from a proximal direction to a distal direction;
a probe (22) being attached to the horn (27) so that when the probe (22) is connected to the horn (27), the ultrasonic vibration is transmitted to a distal face (43) of the probe (22) via the horn (27) and the probe (22), and that the probe (22) and the horn (27) constitute the vibration transmitting portion,
a first tube (55) which extends along the longitudinal axis (C) in the vibration transmitting portion;
a first spacer (56) which contacts an inside of the vibration transmitting portion and an outer circumferential surface of the first tube (55) at a node position (A) of the ultrasonic vibration in a direction parallel to the longitudinal axis (C) and which supports the first tube (55) so that the outer circumferential surface of the first tube (55) does not contact the inside of the vibration transmitting portion;
a second tube (61) disposed inside the first tube (55), extending along the longitudinal axis (C), and permitting a suction target to be suctioned toward the proximal direction; and
a second spacer (64), which contacts an inner circumferential surface of the first tube (55) and an outer circumferential surface of the second tube (61), and which is located off the node position of the ultrasonic vibration such that a clearance is formed between the inner circumferential surface of the first tube (55) and the outer circumferential surface of the second tube (61).

2. The ultrasonic treatment device (11) according to claim 1, wherein the first spacer (56) and the second spacer (64) are lower in heat conductivity than the ultrasonic wave transmitting portion.

3. The ultrasonic treatment device (11) according to claim 1, wherein a first axially parallel dimension of the first spacer (56) in the longitudinal axis (C) direction is smaller than a second axially parallel dimension of the second spacer (64) in the longitudinal axis direction (C).

4. The ultrasonic treatment device (11) according to claim 1, wherein the inner circumferential surface of the second tube (61) has a lower friction property than the inner surface of the vibration transmitting portion.

5. The ultrasonic treatment device (11) according to claim 1, wherein the first tube (55) and the second tube (61) extend in the internal space (52) of the probe (22) and are attachable and detachable from the vibration transmitting portion.

6. The ultrasonic treatment device (11) according to claim 1, wherein the first spacer (56) abuts on the outer circumferential portion of the first tube (55) in part of a range in a direction around the longitudinal axis (C).

7. The ultrasonic treatment device (11) according to claim 1, wherein the first tube (55) and the first spacer (56) are lower in heat conductivity than the vibration transmitting portion, and
the inner circumferential surface of the first tube (55) has a lower frictional property than the inner surface of the vibration transmitting portion.

8. The ultrasonic treatment device (11) according to claim 7, further comprising a ring member (62) which is formed by the integration of the first tube (55) and the first spacer (56) and which abuts on the inner surface circumferentially in a direction around the longitudinal axis (C) and in which a path portion (58) is formed.

9. The ultrasonic treatment device (11) according to claim 8, wherein the ring member (62) is provided over a predetermined range which includes the node position (A) where the first spacer (56) is located in the longitudinal axis (C) direction and which is smaller than a quarter wavelength of the ultrasonic vibration.

## Patentansprüche

1. Ultraschallbehandlungsvorrichtung (11), umfassend:
einen Ultraschallschwinger (26), der eine Ultraschallschwingung erzeugt;
ein Horn (27), das mit dem Ultraschallschwinger (26) gekoppelt ist;
einen Schwingungsübertragungsabschnitt, der sich entlang einer Längsachse (C) erstreckt und der die Ultraschallschwingung von einer proximalen Richtung in eine distale Richtung überträgt;
eine Sonde (22), die an dem Horn (27) befestigt ist, sodass die Ultraschallschwingung, wenn die Sonde (22) mit dem Horn (27) verbunden wird, über das Horn (27) und die Sonde (22) an eine distale Fläche (43) der Sonde (22) übertragen wird und sodass die Sonde (22) und das Horn (27) den Schwingungsübertragungsabschnitt bilden,
ein erstes Rohr (55), das sich entlang der Längsachse (C) in dem Schwingungsübertragungsabschnitt erstreckt;
einen ersten Abstandhalter (56), der eine Innenseite des Schwingungsübertragungsabschnitts und eine äußere Umfangsfläche des ersten Rohrs (55) an einer Knotenposition (A) der Ultraschallschwingung in eine Richtung parallel zu der Längsachse (C) kontaktiert und der das erste Rohr (55) trägt, sodass die äußere Umfangsfläche des ersten Rohrs (55) die Innenseite des Schwingungsübertragungsabschnitts nicht kontaktiert;
ein zweites Rohr (61), das innerhalb des ersten Rohrs (55) angeordnet ist, welches sich entlang der Längsachse (C) erstreckt und das es ermöglicht, dass ein Absaugziel in die proximale Richtung hin abgesaugt wird; und
einen zweiten Abstandhalter (64), der eine innere Umfangsfläche des ersten Rohrs (55) und eine äußere Umfangsfläche des zweiten Rohrs (61) kontaktiert und der sich abseits der Knotenposition der Ultraschallschwingung befindet, sodass zwischen der inneren Umfangsfläche des ersten Rohrs (55) und der äußeren Umfangsfläche des zweiten Rohrs (61) ein Zwischenraum ausgebildet ist.

2. Ultraschallbehandlungsvorrichtung (11) nach Anspruch 1, wobei der erste Abstandhalter (56) und der zweite Abstandhalter (64) eine niedrigere Wärmeleitfähigkeit aufweisen als der Ultraschallübertragungsabschnitt.

3. Ultraschallbehandlungsvorrichtung (11) nach Anspruch 1, wobei eine erste achsparallele Dimension des ersten Abstandhalters (56) in der Längsachsenrichtung (C) kleiner ist als eine zweite achsparallele Dimension des zweiten Abstandhalters (64) in der Längsachsenrichtung (C).

4. Ultraschallbehandlungsvorrichtung (11) nach Anspruch 1, wobei die innere Umfangsfläche des zweiten Rohrs (61) eine geringere Reibungseigenschaft aufweist als die Innenfläche des Schwingungsübertragungsabschnitts.

5. Ultraschallbehandlungsvorrichtung (11) nach Anspruch 1, wobei das erste Rohr (55) und das zweite Rohr (61) sich in dem Innenraum (52) der Sonde (22) erstrecken und an dem Schwingungsübertragungsabschnitt befestigbar und davon abnehmbar sind.

6. Ultraschallbehandlungsvorrichtung (11) nach Anspruch 1, wobei der erste Abstandhalter (56) an den äußeren Umfangsabschnitt des ersten Rohrs (55) in einem Teil eines Bereichs in eine Richtung um die Längsachse (C) herum stößt.

7. Ultraschallbehandlungsvorrichtung (11) nach Anspruch 1, wobei das erste Rohr (55) und der erste Abstandhalter (56) eine niedrigere Wärmeleitfähigkeit aufweisen als der Schwingungsübertragungsabschnitt und
die innere Umfangsfläche des ersten Rohrs (55) eine geringere Reibungseigenschaft aufweist als die Innenfläche des Schwingungsübertragungsabschnitts.

8. Ultraschallbehandlungsvorrichtung (11) nach Anspruch 7, ferner umfassend ein Ringelement (62), das durch die Eingliederung des ersten Rohrs (55) und des ersten Abstandhalters (56) gebildet ist und das in Umfangsrichtung in eine Richtung um die Längsachse (C) herum an die Innenfläche stößt und in dem ein Pfadabschnitt (58) ausgebildet ist.

9. Ultraschallbehandlungsvorrichtung (11) nach Anspruch 8, wobei das Ringelement (62) über einen vorgegebenen Bereich bereitgestellt ist, der die Knotenposition (A) beinhaltet, in welcher der erste Abstandhalter (56) sich in der Längsachsenrichtung (C) befindet und der kleiner ist als eine Viertelwellenlänge der Ultraschallschwingung.

## Revendications

1. Dispositif de traitement par ultrasons (11) comprenant :
un vibreur ultrasonore (26) générant une vibration ultrasonore ;
un cornet (27) couplé au vibreur ultrasonore (26) ;
une partie de transmission de vibration qui s'étend le long d'un axe longitudinal (C) et qui transmet la vibration ultrasonore depuis une direction proximale vers une direction distale ;
une sonde (22) qui est fixée au cornet (27) de telle sorte que, lorsque la sonde (22) est reliée au cornet (27), la vibration ultrasonore est transmise à une face distale (43) de la sonde (22) par l'intermédiaire du cornet (27) et de la sonde (22), et que la sonde (22) et le cornet (27) constituent la partie de transmission de vibration,
un premier tube (55) qui s'étend le long de l'axe longitudinal (C) dans la partie de transmission de vibration ;
une première entretoise (56) qui est en contact avec l'intérieur de la partie de transmission de vibration et une surface circonférentielle externe du premier tube (55) au niveau d'une position de noeud (A) de la vibration ultrasonore dans une direction parallèle à l'axe longitudinal (C) et qui porte le premier tube (55) de telle sorte que la surface circonférentielle externe du premier tube (55) n'est pas en contact avec l'intérieur de la partie de transmission de vibration ;
un second tube (61) disposé à l'intérieur du premier tube (55), s'étendant le long de l'axe longitudinal (C), et permettant à une cible d'aspiration d'être aspirée vers la direction proximale ; et
une seconde entretoise (64), qui est en contact avec une surface circonférentielle interne du premier tube (55) et une surface circonférentielle externe du second tube (61), et qui se trouve hors de la position de noeud de la vibration ultrasonore de telle sorte qu'un jeu est formé entre la surface circonférentielle interne du premier tube (55) et la surface circonférentielle externe du second tube (61).

2. Dispositif de traitement par ultrasons (11) selon la revendication 1, dans lequel la première entretoise (56) et la seconde entretoise (64) ont une conductivité thermique inférieure à celle de la partie de transmission d'onde ultrasonore.

3. Dispositif de traitement par ultrasons (11) selon la revendication 1, dans lequel une première dimension axialement parallèle de la première entretoise (56) dans la direction de l'axe longitudinal (C) est plus petite qu'une seconde dimension axialement parallèle de la seconde entretoise (64) dans la direction de l'axe longitudinal (C).

4. Dispositif de traitement par ultrasons (11) selon la revendication 1, dans lequel la surface circonférentielle interne du second tube (61) possède une propriété de frottement inférieure à celle de la surface interne de la partie de transmission de vibration.

5. Dispositif de traitement par ultrasons (11) selon la revendication 1, dans lequel le premier tube (55) et le second tube (61) s'étendent dans l'espace interne (52) de la sonde (22) et peuvent être fixés à et détachés de la partie de transmission de vibration.

6. Dispositif de traitement par ultrasons (11) selon la revendication 1, dans lequel la première entretoise (56) est en butée contre la partie circonférentielle externe du premier tube (55) dans une partie d'une plage dans une direction autour de l'axe longitudinal (C).

7. Dispositif de traitement par ultrasons (11) selon la revendication 1, dans lequel le premier tube (55) et la première entretoise (56) ont une conductivité thermique inférieure à celle de la partie de transmission de vibration, et
la surface circonférentielle interne du premier tube (55) a une propriété de frottement inférieure à celle de la surface interne de la partie de transmission de vibration.

8. Dispositif de traitement par ultrasons (11) selon la revendication 7, comprenant en outre un élément d'anneau (62) qui est formé par l'intégration du premier tube (55) et de la première entretoise (56) et qui est en butée contre la surface interne de manière circonférentielle dans une direction autour de l'axe longitudinal (C), et dans lequel une partie de trajet (58) est formée.

9. Dispositif de traitement par ultrasons (11) selon la revendication 8, dans lequel l'élément d'anneau (62) est placé dans une plage prédéterminée qui comprend la position de noeud (A) dans laquelle la première entretoise (56) est située dans la direction de l'axe longitudinal (C) et qui est plus petite qu'un quart de longueur d'onde de la vibration ultrasonore.
